# NEUE EUROPÄISCHE PATENTSCHRIFT

(11) **EP 0 839 847 B2**
(45) Veröffentlichungstag und Bekanntmachung des Hinweises auf die Entscheidung über den Einspruch: **09.06.2004**
(45) Hinweis auf die Patenterteilung: 24.11.1999
(21) Anmeldenummer: 97115394.5
(22) Anmeldetag: 05.09.1997
(51) Int. Cl.: C08G 18/80, C08G 18/70, C08G 18/08

(54) **Stabile und feststoffreiche wässrige Dispersionen von blockierten Polyisocyanaten**
High solids and stable aqueous dispersions of blocked polyisocyanates
Dispersions de polyisocyanates bloqués stables et à haute teneur en solide

(30) Priorität: 29.10.1996 DE 19644838
(43) Veröffentlichungstag der Anmeldung: 06.05.1998
(73) Patentinhaber: Degussa AG, 40474 Düsseldorf (DE)
(72) Erfinder: Lange, Hartwig, Dr., 45721 Haltern (DE); Janischewski, Klaus, 46284 Dorsten (DE); Reichel, Dirk, Dr., 46284 Dorsten (DE)

(56) Entgegenhaltungen:
- EP-A- 0 012 348
- EP-A- 0 075 775
- EP-A- 0 312 836
- EP-A- 0 524 511
- EP-A- 0 537 578
- US-A- 4 098 933

## Beschreibung

Die Erfindung betrifft stabile und festoffreiche, hilfslösemittelhaltige oder praktisch hilfslösemittelfreie wäßrige Dispersionen von blockierten Polyisocyanaten, ein Verfahren zur Herstellung solcher Dispersionen sowie die Verwendung solcher Dispersionen als Vernetzer für Lackharze.

### 1. Stand der Technik

Blockierte (oder verkappte) Polyisocyanate sind bekannte und in großem Umfang verwendete Vernetzungsmittel für Lackharze in lösemittelhaltigen Beschichtungssystemen. Aus Gründen des Umweltschutzes und natürlich auch aus Kostengründen streben viele technische Entwicklungen der jüngeren Vergangenheit eine Verminderung der Lösemittelmenge oder, was besonders erwünscht ist, lösemittelfreie oder praktisch lösemittelfreie Systeme an.

Wäßrige Dispersionen von blockierten Polyisocyanaten eignen sich zur Verwendung in Einkomponenten-Wasserlacken (oder wasserverdünnbaren Lakken), d.h. in wäßrigen, lösemittelfreien oder praktisch lösemittelfreien Beschichtungssystemen, die neben den als Vernetzungsmittel fungierenden blockierten Polyisocyanaten ein in Wasser gelöstes oder dispergiertes Lackharz mit funktionalen Gruppen enthalten, die mit Isocyanatgruppen zu reagieren vermögen. Da blokkierte Isocyanate in den meisten Fällen Feststoffe oder hochviskose Substanzen sind, können sie in Wasser nur unter Zusatz von Lösemitteln oder in Substanz nur oberhalb ihres Schmelzpunkts dispergiert werden. Die Nachteile der beiden Verfahren liegen darin, daß im ersteren Fall das zur Verdünnung eingesetzte Lösemittel nach der Dispergierung unter erheblichem Aufwand wieder entfernt werden muß, während im Fall der Schmelzemulgierung bei den erforderlichen hohen Temperaturen schon ein Teil des Blockierungsmittels abspaltet, die freigesetzten Isocyanatgruppen bei der Dispergierung mit Wasser reagieren und nicht mehr zur Vernetzung mit den funktionalen Gruppen des Lackharzes zur Verfügung stehen.

Die wäßrigen Dispersionen der blockierten Polyisocyanate (und natürlich auch die der Lackharze) sollten möglichst feststoffreich sein. Der wichtigste Vorteil feststoffreicher Dispersionen besteht darin, daß man mit nur einem Auftrag dicke Schichten mit hervorragenden Eigenschaften (Fülle, Beständigkeit, Deckvermögen) erzeugen kann. Dies bedeutet gegenüber mehrschichtigem Auftrag eine Zeit- und (wegen der mehrfachen Einbrennvorgänge) Energieersparnis, aber auch eine Qualitätsoptimierung, denn jede zusätzliche Schicht erhöht das Risiko von Fehlproduktionen. Daneben mindern feststoffreiche Dispersionen auch die Kosten für Transport und Lagerung. Die Dispersionen sollten zudem stabil sein, d.h. lange lagerfähig, ohne in erheblichem Umfang zu koagulieren oder zu sedimentieren. Soweit trotzdem Koagulation oder Sedimentation eintritt, sollten die Abscheidungen leicht und nachhaltig redispergierbar sein.

Bekannte Verfahren zur Herstellung stabiler wäßriger Dispersionen von blockierten Polyisocyanaten benutzen externe Emulgatoren und Schutzkolloide (JP 4 216 815, JP 4 216 816, JP 4 216 817 und WO 94/22935). Ein Nachteil dieser Verfahren ist die permanente Hydrophilie der Emulgatoren und der Schutzkolloide, die in den Beschichtungen erhalten bleibt, welche beim Einbrennen durch Reaktion der Polyisocyanate mit den Isocyanat-reaktive Gruppen der Lackharze entstehen. Diese Beschichtungen neigen daher dazu, bei Einwirkung von Wasser einzutrüben, zu erweichen und/oder aufzuquellen, was die Verwendung solcher Dispersionen zumindest für Lacke ausschließt, die im Außenbereich eingesetzt werden sollen.

Das in DE 24 56 469 beschriebene Verfahren zur Herstellung von wäßrigen Dispersionen geht von teilblockierten Polyisocyanaten aus, die mit einem Hydrophilierungsmittel umgesetzt werden, das eine NCO-reaktive Gruppe sowie eine hydrophile oder potentiell hydrophile Gruppe, z.B. eine Sulfosäure- oder Carboxylgruppe, enthält. Beide Arten von Gruppen können durch Neutralisation in ionisch hydrophile Gruppen, z. B. Sulfonat- oder Carboxylatgruppen, umgewandelt werden. In den Beispielen werden als Hydrophilierungsmittel N-Methylaminoethansulfonsäure und Aminoessigsäure in Form ihrer Natriumsalze verwendet. Das Umsetzungsprodukt wird dann in Wasser dispergiert. Ein ähnliches Verfahren wird in EP 0 012 348 beschrieben, wobei die blockierten hydrophilierten Polyisocyanate nicht nur selbstdispergierbar sind, sondern auch die Dispergierung von hydrophoben Lackharzen fördern. Bei diesen wirtschaftlich nicht genutzten Verfahren erfolgt jedoch die Anknüpfung der Verbindung mit (potentiell) hydrophiler Gruppe infolge der vorherigen Teilblockierung bei niedrigen NCO-Gehalten, was einerseits eine unwirtschaftlich lange Reaktionszeit erfordert und andererseits zur Folge hat, daß entweder nicht alle freien NCO-Gruppen hydrophil verknüpft werden oder, bei Überschuß an Hydrophilierungsmittel, nicht umgesetztes Hydrophilierungsmittel in der Polyisocyanatdispersion verbleibt.

In DE 27 08 611 wird ein Verfahren zur Herstellung von Polyurethan-Präpolymeren beschrieben, bei dem man Polyisocyanate zunächst mit einem Unterschuß an Hydroxycarbonsäuren, wie Dimethylolpropionsäure, umsetzt und dann die frei gebliebenen Isocyanatgruppen blokkiert. Diese Polyurethan-Präpolymeren müssen hohe Säurezahlen von >30 mg KOH/g aufweisen, da bei niedrigeren Säurezahlen stabile Dispersionen oder Lösungen nur mit zusätzlichen organischen Lösemitteln herzustellen sind. Nachteilig bei diesem Verfahren ist, daß ein sehr erheblicher Teil der ursprünglichen Isocyanatgruppen durch die Umsetzung mit dem Hydrophilierungsreagens Hydroxycarbonsäure für die Vernetzung mit dem Lackharz verlorengeht. Ein weiterer Nachteil dieses Verfahrens besteht in der hohen Viskosität von Dispersionen mit >35 Gew.-% Feststoffanteil, die die Verarbeitung erschwert oder praktisch unmöglich macht.

Die EP-0 312 836 beschreibt ein Verfahren, bei dem Isocyanatgruppen durch Reaktion mit einem Cyanharnstoffsalz verkappt werden. Die so geschaffenen hydrophilen Gruppen nehmen an der Vernetzungsreaktion beim Einbrennen teil, beeinträchtigen also nicht die Vernetzungsdichte. Ein Nachteil ist aber, daß beim Einbrennen Amine abgespalten werden, die den Lackfilm nur schwer verlassen und bei hellen Lacken eine störende Vergilbung verursachen.

In EP 0133 223 werden mittels cyclischer Malonate hydrophilierte blockierte Polyisocyanate beschrieben. In diesen Systemen erfolgt die Härtung ebenfalls unter Mitwirkung der cyclischen Malonatgruppen, so daß durch die hydrophile Modifizierung wiederum keine Verminderung der Vernetzungsdichte eintritt. Nachteilig bei diesem Verfahren sind aber die erforderlichen erheblichen Mengen an wasserlöslichem organischen Lösemittel.

Aus EP-A1-0 524 511 ist ein Verfahren bekannt, bei dem nichtionisch modifizierte Polyisocyanate mit freien Isocyanatgruppen erst in Wasser dispergiert und dann die Isocyanatgruppen blockiert werden. Dabei reagiert jedoch ein Teil der Isocyanatgruppen mit Wasser und geht für die Vernetzungsreaktion verloren. Außerdem ist es schwierig, die jeweils gewünschten stöchiometrischen Verhältnisse reproduzierbar einzustellen.

Weiterhin ist aus EP-A2-0 537 578 ein Verfahren bekannt, nach dem blockierte Polyisocyanate für die Textilveredelung (Knitterfestausrüstung) u.a. mit eingebauten Polyalkylenoxideinheiten hydrophiliert und dispergiert werden. Auch in EP 424 697 und DE 28 14 815 werden Dispersionen von blockierten nichtionisch hydrophilierten Polyisocyanaten beschrieben. Diese Dispersionen sind aber wiederum permanent hydrophil, so daß mit ihnen hergestellte Beschichtungen bei Feuchtigkeitseinwirkung eintrüben, erweichen und/oder aufquellen können und daher für Außenanwendungen ungeeignet sind.

In EP 0 022 452 wird ein Einkomponenten-Polyurethansystem beschrieben, das als Härter ein Polyurethan-Präpolymer enthält, bei dem jedes Molekül mit einem hydrophilen Modifizierungsreagens verknüpft ist. Neben dem Verlust an vernetzungsfähigen Isocyanatgruppen ist hier von Nachteil, daß ein Lösemittelgemisch aus Wasser und Alkohol erforderlich ist und vor allem daß Dispersionen mit einem Feststoffanteil von >40 Gew.-% eine so hohe Viskosität aufweisen, daß sie schwierig zu verarbeiten sind.

Schließlich beschreiben EP 0 566 953 und EP 0 576 952 spezielle mittels Hydroxycarbonsäuren hydrophilierte Polyisocyanatgemische, die für die Härtung von Lackharzen in wäßrigen Systemen geeignet sind.

### 2. Beschreibung der Erfindung

Einer der Gegenstände der Erfindung ist ein Verfahren zur Herstellung stabiler und feststoffreicher wäßriger, hilfslösemittelhaltiger Dispersionen von blokkierten Polyisocyanaten, bei dem man
(1) eine Lösung eines Polyisocyanatgemisches aus 20 bis 70 Gew.-% eines blockierten hydrophil modifizierten Polyisocyanats (A) und 30 bis 80 Gew.-% eines blockierten hydrophoben Polyisocyanats (B) in einem Hilfslösemittel (G) herstellt und
(2) dieser Lösung unter intensivem Mischen soviel Wasser zusetzt, daß ein zweiphasiges System aus einer dispersen Polyisocyanatphase und einer kontinuierlichen wäßrigen, hilfslösemittelhaltigen Phase entsteht.

Bei einer vorteilhaften Ausführungsform dieses Verfahrens stellt man entsprechende Dispersionen her, die jedoch praktisch frei von Hilfslösemittel (G) sind, indem man
(3) der Lösung der Stufe (1) eine erste Teilmenge des insgesamt verwendeten Wassers zusetzt,
(4) das Hilfslösemittel (G) unter intensivem Mischen aus dem entstehenden wäßrigen, hilfslösemittelhaltigen Gemisch weitgehend entfernt und
(5) dem verbleibenden Gemisch aus den blockierten Polyisocyanaten (A) und (B) und der ersten Teilmenge Wasser eine zweite Teilmenge Wasser zusetzt,
mit der Maßgabe, daß (i) die erste Teilmenge Wasser so bemessen wird, daß in dem Gemisch, das nach der weitgehenden Entfernung des Hilfslösemittels (G) verbleibt, die Polyisocyanate (A) und (B) die kontinuierliche Phase bilden und (ii) die Zugabe der zweiten Teilmenge Wasser zumindest bis zur Phasenumkehr unter intensivem Mischen erfolgt.

Ein anderer Gegenstand der Erfindung sind stabile und feststoffreiche wäßrige, hilfslösemittelhaltige und insbesondere praktisch hilfslösemittelfreie Dispersionen von blockierten Polyisocyanaten, die als disperse Phase ein Polyisocyanatgemisch aus 20 bis 70 Gew.-% eines blockierten hydrophil modifizierten Polyisocyanats (A) und 30 bis 80 Gew.-% eines blockierten hydrophoben Polyisocyanats (B) enthalten, wie sie nach dem Verfahren der Erfindung und insbesondere nach dessen vorteilhafter Ausführungsform erhältlich sind.

Schließlich ist ein Gegenstand der Erfindung die Verwendung der erfindungsgemäßen stabilen und feststoffreichen Dispersionen der blockierten Polyisocyanate (A) und (B) als Vernetzer für Lackharze.

### 2.1 Eigenschaften und Vorteile der Dispersionen nach der Erfindung

Das Verfahren nach der Erfindung ergibt überraschenderweise Dispersionen mit einer optimalen Kombination vorteilhafter Eigenschaften:
- Der Feststoffgehalt ist hoch. Er beträgt bei den hilfslösemittelhaltigen Systemen in der Regel mindestens 35 und bis zu 80 Gew.-%, vorteilhaft 35 bis 60 Gew.-%. Bei den praktisch hilfslösemittelfreien Systemen liegt er im allgemeinen im Bereich von mindestens 35 Gew.-% bis zu 60 Gew.-%, vorteilhaft von 40 bis 50 Gew.-%.
- Die Viskositäten der feststoffreichen Dispersionen bei 25°C betragen trotz des hohen Feststoffgehalts in der Regel <1.000 mPa·s, so daß sie sich problemlos verarbeiten lassen.
- Der latente Isocyanatgehalt (d.h. der Gewichtsanteil an Isocyanatgruppen - unblockiert gerechnet - an dem blockierten Polyisocyanatgemisch aus (A) und (B)) ist trotz der Hydrophilierung hinreichend hoch. Er beträgt im allgemeinen >8 Gew.-% und kann bis zu 15 Gew.-% betragen. Die mittlere latente NCO-Funktionalität liegt, je nach eingesetztem Polyisocyanat. in der Regel im Bereich von 2,5 bis 4,5 und kann bis zu 6,0 und mehr betragen, so daß die besonders erwünschte hohe Vernetzungsdichte der eingebrannten Beschichtungen gewährleistet ist. Der hohe latente Isocyanatgehalt und die hohe Vernetzungsdichte sorgen für hervorragende mechanische Eigenschaften, wie Härte, sowie für Chemikalienbeständigkeit der vernetzten Beschichtungen. Insoweit sind die mit den Dispersionen nach der Erfindung hergestellten Beschichtungen den entsprechenden Beschichtungen nach dem Stand der Technik mindestens ebenbürtig.
- Bei Verwendung niedrigsiedender Hilfslösemittel (G) lassen sich diese bei der bevorzugten Ausführungsform der Erfindung nach dem Zusatz einer ersten Teilmenge des insgesamt verwendeten Wassers weitgehend abdestillieren, wodurch der Gehalt der fertigen Dispersion, wie sie durch Zugabe der zweiten Teilmenge Wasser entsteht, an restlichem Hilfslösemittel (G) unschwer auf <2 Gew.-%, insbesondere <0,5 Gew.-% abgesenkt werden kann. Die Dispersionen sind also praktisch hilfslösemittelfrei. Gehalte an restlichem Hilfslösemittel (G) von <0,5 Gew.-% sind vorteilhaft, weil sie keinen unangenehmen Geruch bei der Verarbeitung verursachen.
- Die Dispersionen nach der Erfindung sind sowohl bei Raumtemperatur, als auch bei leicht erhöhter Temperatur bis zu 60°C über lange Zeiträume lagerfähig. Beispielsweise scheiden praktisch hilfslösemittelfreie Dispersionen mit 45 bis 50 Gew.-% Feststoffanteil unter optimalen Bedingungen innerhalb von 6 Monaten bei Raumtemperatur oder innerhalb von 4 bis 8 Wochen bei 60°C <1 Gew.-% Feststoff ab. Die geringen Abscheidungen sind zudem in der Regel leicht und nachhaltig redispergierbar. Diese gute bis hervorragende Langzeitbeständigkeit wird mit einem Minimum an Hydrophilie, nämlich durch Hydrophilierung nur eines Teils des Polyisocyanats, ohne Zusatz eines externen Dispergiermittels und bzw. oder Schutzkolloids sowie ohne Einbau von hydrophilierende Polyoxyalkyleneinheiten und - zumindest bei den bevorzugten Dispersionen - auch ohne Lösemittelzusatz erreicht.
- Die minimale Hydrophilie hat zudem die erwünschte Folge, daß die ausgehärteten Beschichtungen wenig empfindlich gegen Feuchtigkeit sind und sich daher gut auch für Außenanwendungen eignen.

Es ist ein wesentliches Merkmal der Erfindung, daß das blockierte Polyisocyanat ein Gemisch aus 20 bis 70 Gew.-% eines blockierten hydrophil modifizierten Polyisocyanats (A) und 30 bis 80 Gew.-% eines blokkierten hydrophoben Polyisocyanat (B) ist. Die hydrophil modifizierten Polyisocyanate (A) erleichtern offenbar die Dispergierung der hydrophoben Polyisocyanate (B) zu Dispersionen mit anwendungstechnisch optimalen Eigenschaften, wie Viskosität, Teilchengröße und Härte der späteren Lackfilme, und stabilisieren die Dispersionen nachhaltig. Vorteilhaft beträgt der Anteil des blokkierten hydrophil modifizierten Polyisocyanats (A) 35 bis 60 Gew.-% und insbesondere 40 bis 50 Gew.-%. Der Anteil des blockierten hydrophoben Polyisocyanats (B) beträgt demzufolge vorteilhaft 40 bis 65 Gew.-% und insbesondere 50 bis 60 Gew.-%.

### 2.2 Die hydrophil modifizierten Polyisocyanate (A) und die hydrophoben Polyisocyanate (B)

Die blockierten hydrophil modifizierten Polyisocyanate (A) und ebenso die blockierten hydrophoben Polyisocyanate (B) leiten sich von den üblichen in der Lackchemie verwendeten hydrophoben Polyisocyanaten (C) ab. Statt nur eines blockierten hydrophil modifizierten Polyisocyanats (A) und/oder nur eines blockierten hydrophoben Polyisocyanats (B) kann man nicht selten mit Vorteil Polyisocyanate (A) und/oder (B) einsetzen, die auf Gemische verschiedener hydrophober Polyisocyanate (C) zurückgehen. Zu hydrophil modifizierten Polyisocyanaten (D) werden die hydrophoben Polyisocyanate (C) dadurch, daß sie mit einem Hydrophilierungsmittel (E) und gegebenenfalls einem Neutralisierungsmittel (H), wie später erläutert, umgesetzt werden. Durch Umsetzung mit einem Blockierungsmittel (F) wird das hydrophil modifizierte Polyisocyanat (D) zu dem blockierten hydrophil modifizierten Polyisocyanat (A) und das hydrophobe Polyisocyanat (C) zu dem blokkierten hydrophoben Polyisocyanat (B).

Grundsätzlich eignen sich für die Erfindung alle aliphatischen, cycloaliphatischen, araliphatischen, aromatischen oder heterocyclischen Polyisocyanate, wie sie z.B. von W.Siefken in Liebigs Annalen der Chemie, 562, Seiten 75 bis 136, beschrieben sind. Eine ausführliche Darstellung der einsetzbaren Polyisocyanate findet sich in EP 0 537 578, Seite 3, Zeilen 10 bis 45. Bevorzugte Polyisocyanate sind, wegen der hohen Licht- und Wetterbeständigkeit der damit hergestellten Lackfilme, aliphatische und/oder cycloaliphatische Polyisocyanate mit einem mittleren Molekulargewicht von bis zu etwa 1.000 g/mol, vorteilhaft von etwa 800 g/mol, und einer mittleren Isocyanatfunktionalität von 2 bis 4. Dazu gehören beispielsweise einfache Diisocyanate, wie 1,6-Diisocyanatohexan (HDI), Bis(4-isocyanatocyclohexyl )methan (HMDI), 1,5-Diisocyanato-2-methylpentan (MPDI), 1,6-Diisocyanato-2,4,4-trimethylhexan und/oder 1,6-Diisocyanato-2,2,4-trimethylhexan (TMDI) sowie insbesondere 3-Isocyanatomethyl-3,5,5-trimethylcyclohexylisocyanat (Isophorondiisocyanat; IPDI). Weiterhin gehören dazu durch katalytische Umsetzung dieser einfachen Diisocyanate, vornehmlich von IPDI, HDI und/oder HMDI, hergestellte Dimere mit Uretdion-Struktur. Eine andere bevorzugte Klasse von Polyisocyanaten sind die durch Allophanatisierung, Trimerisierung, Biuretisierung oder Urethanisierung der einfachen Diisocyanate hergestellten Verbindungen mit mehr als zwei Isocyanatgruppen pro Molekül, beispielsweise die Umsetzungsprodukte dieser einfachen Diisocyanate, wie IPDI, HDI und/oder HMDI, mit mehrwertigen Alkoholen (z.B. Glycerin, Trimethylolpropan, Pentaerythrit) bzw. mehrwertigen Polyaminen oder die Triisocyanurate, die durch Trimerisierung der einfachen Diisocyanate, wie IPDI, HDI und HMDI, erhältlich sind. Als Vertreter der (weniger bevor- zugten) aromatischen Polyisocyanate seien z.B. 2,4-und/oder 2,6-Diisocyanatoluol sowie 4,4'- und/oder 2,4'-Diisocyanatodiphenylmethan erwähnt.

### 2.3 Das Hydrophilierungsmittel (E)

Wie erwähnt, können die hydrophoben Polyisocyanate (C) durch Umsetzung mit einem Hydrophilierungsmittel (E) und gegebenenfalls mit einem Neutralisierungsmittel (H), wie später erläutert, in hydrophil modifizierte Polyisocyanate (D) umgewandelt werden. Geeignete Hydrophilierungsmittel (E) enthalten mindestens eine NCO-reaktive Gruppe mit einem nach dem Zerewitinoff-Test aktiven Wasserstoffatom und mindestens eine hydrophile oder potentiell hydrophile Gruppe. Von den NCO-reaktiven Gruppen seien insbesondere Hydroxylgruppen sowie primäre und sekundäre Aminogruppen genannt. Hydrophile Gruppen können u.a. wiederum Hydroxylgruppen sein, die in dem Hydrophilierungsmittel vorhanden sein oder aus im Hydrophilierungsmitel vorhandenen Gruppen entstehen können. Bevorzugte hydrophile Gruppen sind z.B. die Sulfosäure- und die Phosphonsäuregruppe. Diese sind gegenüber der Carboxylgruppe vergleichsweise stark dissoziiert und werden daher als hydrophil angesehen. Sie können durch Neutralisation in die ionisch hydrophilen Sulfonat- bzw. Phosphonatgruppen umgewandelt werden, wodurch der pH-Wert der Dispersion in den neutralen oder basischen Bereich angehoben wird, was in der Regel empfehlenswert ist. Die Carboxylgruppe ist ein Beispiel für eine potentiell hydrophile Gruppe, weil sie nur schwach dissoziiert und daher kaum hydrophil ist, aber durch Neutralisierung praktisch vollständig in die stark dissoziierte, ionisch hydrophile Carboxylatgruppe umgewandelt wird. Ein anderes Beispiel für potentiell hydrophile Gruppen ist die tertiäre Aminogruppe, die durch Umsetzung (oder Neutralisierung) mit einer Säure in die ionisch hydrophile quaternäre Ammoniumgruppe umgewandelt wird. Das Hydrophilierungsmittel (E) darf natürlich nur in einer solchen Menge angewandt werden, daß genügend Isocyanatgruppen für die Vernetzungsreaktion mit dem Lackharz erhalten bleiben.

Eine ausführliche Darstellung der Hydrophilierungsmittel findet sich in EP 0 537 578. Geeignete Hydrophilierungsmittel (E) sind z.B. Hydroxycarbonsäuren, Hydroxysulfonsäuren, Hydroxyphosphonsäuren, Aminocarbonsäuren, Aminosulfonsäuren und tertiäre Aminoalkanole. Im einzelnen seien beispielsweise Glykolsäure, beta-Aminopropionsäure, Hydroxyethansulfonsäure, Hydroxyethanphosphonsäure und N.N-Dimethylaminoethanol genannt. Ein besonders geeignetes Hydrophilierungsmittel (E) ist Dimethylolpropionsäure (DMPS), deren Hydroxylgruppen mit jeweils einer Isocyanatgruppe reagieren und die dadurch zwei Moleküle Polyisocyanat unter Erhöhung der Molmasse und gegebenenfalls auch der NCO-Funktionalität verknüpfen kann. Die Carboxylgruppe verhält sich unter den Reaktionsbedingungen, wohl infolge sterischer Hinderung, gegenüber den Isocyanatgruppen praktisch inert.

Statt ein hydrophobes Polyisocyanat (C) zu einem hydrophil modifizierten Polyisocyanat (D) umzusetzen und dieses zur gemeinsamen Blockierung mit weiterem hydrophoben Polyisocyanat (C) zu mischen, kann man das Gemisch der Polyisocyanate (A) und (B) auch dadurch erzeugen, daß man eine diesem Gemisch entsprechende Menge hydrophobes Polyisocyanat (C) nur teilweise hydrophiliert, indem man sie mit einer solchen Menge an Hydrophilierungsmittel (E) umsetzt, daß die Anteile an entstehendem hydrophil modifizierten Polyisocyanat (D) und an verbliebenem hydrophoben Polyisocyanat (C) dem für die fertige Dispersion angestrebten Verhältnis von blockiertem hydrophil modifizierten Polyisocyanat (A) zu blockiertem hydrophoben Polyisocyanat (B) entsprechen, gegebenenfalls potentiell hydrophile Gruppen durch Neutralisieren in ionisch hydrophile Gruppen umwandelt und die nicht umgesetzten Isocyanatgruppen des Hydrophilierungsgemisches blockiert.

### 2.4 Die Blockierung des hydrophil modifizierten Polyisocyanats (A) und des hydrophoben Polyisocyanats (B)

Die Isocyanatgruppen des hydrophil modifizierten Polyisccyanats (D) und des hydrophoben Polyisocyanats (C) werden in üblicher Weise, jeweils für sich oder bevorzugt gemeinsam, durch Umsetzung mit geeigneten Blockierungsmitteln (F) blockiert. Die Blockierung ist z.B. beschrieben in Progress in Organic Coatings, 3 (1975), Seiten 73 bis 99 sowie in Progress in Organic Coatings 9 (1981), Seiten 3 bis 28. Man verwendet die bekannten Blockierungsmittel (F), die bei 20 bis 120°C mit Isocyanatgruppen eine Additionsreaktion eingehen, die bei höheren Temperaturen reversibel ist, so daß die dann wieder frei werdenden Isocyanatgruppen mit reaktionsfähigen Gruppen eines Lackharzes reagieren können. Geeignete Blockierungsmittel (F) sind z.B. sekundäre oder tertiäre Alkohole, Phenole, C-H-acide Verbindungen (wie Malonsäurederivate), Lactame (z.B. ε-Caprolactam) und Oxime. Bevorzugte Blockierungsmittel sind Oxime, wie Formaldoxim, Acetaldoxim, Cyclohexanonoxim, Acetophenonoxim, Benzophenonoxim und insbesondere Methylethylketoxim. Weitere brauchbare Blockierungsmittel sind 3,5-Dimethylpyrazol und 1,2,4-Triazol.

### 2.5 Das Neutralisierungsmittel (H)

Wenn durch das Hydrophilierungsmittel (E) eine potentiell hydrophile Gruppe, z.B. eine Carboxylgruppe oder eine tertiäre Aminogruppe, in das Polyisocyanat eingeführt wird, so kann diese durch das Neutralisierungsmittel (H) in eine ionisch hydrophile Gruppe umgewandelt werden. Das Neutralisierungsmittel ist eine Base, wenn die potentiell hydrophile Gruppe ein saure Gruppe, z.B. die Carboxylgruppe, ist, und es ist ein Säure im Falle von basischen potentiell hydrophilen Gruppen, z.B. einer tertiären Aminogruppe. Die Basen können anorganische Basen, wie Ammoniak oder Hydrazin, oder organische Basen sein. Bevorzugt werden Ammoniak sowie primäre, sekundäre oder tertiäre Amine, wie Ethylamin, n-Propylamin, Dimethylamin, Di-n-butylamin, Cyclohexylamin, Benzylamin, Morpholin, Piperidin und Triethanolamin. Besonders bevorzugt werden wegen ihres inerten Verhaltens gegenüber den blockierten NCO-Funktionen tertiäre Amine, wie N,N-Dimethylethanolamin, N,N-Diethylaminoethanol, Triethylamin, Tri-npropylamin und Tri-n-butylamin. Geeignete Säuren sind zweckmäßig Carbonsäuren, wie Ameisensäure, Essigsäure und Benzoesäure.

### 2.6 Das Hilfslösemittel (G)

Als Hilfslösemittel (G) werden niedrigsiedende inerte Lösemittel bevorzugt, die mit Wasser zumindest über weite Bereiche keine Mischungslücke bilden, einen Siedepunkt bei Atmosphärendruck von <100°C haben und sich daher gewünschtenfalls durch Destillation leicht bis auf einen Restgehalt von <2 Gew.-% und insbesondere von <0,5 Gew.-%, bezogen auf die fertige Dispersion, abtrennen und wiederverwenden lassen. Geeignete Lösemittel dieser Art sind z.B. Aceton, Methylethylketon oder Tetrahydrofuran. Grundsätzlich geeignet sind auch höhersiedende Lösemittel, wie n-Butylglykol, Di-n-butylglykol und N-Methylpyrrolidon, die in der wasserverdünnbaren Dispersion verbleiben. Sie werden jedoch weniger bevorzugt, weil es ein besonderer Vorteil der Erfindung ist, daß wäßrige Dispersionen hergestellt werden können, die auch ohne umweltbelastende organische Lösemittel feststoffreich und stabil sind.

### 2.7 Das Herstellverfahren

Zunächst wird hydrophobes Polyisocyanat (C) mit dem Hydrophilierungsmittel (E), gegebenenfalls in Anwesenheit eines Katalysators, umgesetzt. Zweckmäßig wendet man das hydrophobe Polyisocyanat (C) als Lösung in einem Hilfslösemittel (G) mit einem Feststoffgehalt von etwa 40 bis 80 Gewichts-% an. Geeignete Katalysatoren sind u.a. organische Zinnsalze, wie Dibutylzinndiacetat oder Dibutylzinndilaurat. Man arbeitet z. B. zunächst bei Raumtemperatur und erhöht die Temperatur zur Vervollständigung der Reaktion bis auf 120°C.

Solche relativ hohen Temperaturen können insbesondere dann erforderlich sein, wenn man die Blockierung in Substanz durchführt. Bei Mitverwendung eines Hilfslösemittels (G) mit entsprechendem Siedepunkt kann man das Reaktionsgemisch einige Zeit am Rückfluß erhitzen. Zweckmäßig arbeitet man bei 40 bis 100°C. Bei diesen Temperaturen sind die potentiell hydrophilen Gruppen des Hydrophilierungsmittels (E) gegenüber der Isocyanatgruppe in der Regel inert oder praktisch inert.

Das Hydrophilierungsmittel wird vorteilhaft in einer solchen Menge angewandt, daß im Mittel auf ein Polyisocyanatmolekül nicht mehr als eine für die Verknüpfung vorgesehene NCO-reaktive Funktion des Hydrophilierungsmittels entfällt. Im Falle der Dimethylolpropionsäure wird also auf 2 Mol Polyisocyanat höchstens 1 Mol der Säure eingesetzt. Vorteilhaft wendet man etwa 1 Äquivalent der für die Verknüpfung vorgesehenen NCO-reaktiven Funktion pro Mol Polyisocyanat an. In jedem Fall entsteht ein Gemisch mit statistischer Verteilung der hydrophilierenden Gruppen. Dieses Gemisch wird als ein hydrophil modifiziertes Polyisocyanat (D) im Sinne der Erfindung betrachtet, auch wenn es nicht hydrophil modifizierte Anteile enthält.

Zur Blockierung der Isocyanatfunktionen kann man eine Mischung aus dem so erhaltenen hydrophil modifizierten Polyisocyanat (D) und hydrophobem Polyisocyanat (C), letzteres gegebenenfalls wiederum als etwa 40- bis 80 gewichtsprozentige Lösung in einem Hilfslösemittel (G), im angegebenen Mengenverhältnis mit dem Blockierungsmittel (F) behandeln. Wie erwähnt, kann die zu blockierende Mischung auch durch Umsetzung von hydrophobem Polyisocyanat (C) mit entsprechendem Unterschuß an Hydrophilierungsmittel (E) und gegebenenfalls mit Neutralisierungsmittel (H) erzeugt werden. Alternativ, aber weniger praktisch, kann man das hydrophil modifizierte Polyisocyanat (D) und das hydrophobe Polyisocyanat (C) jeweils für sich blockieren und die blockierten Produkte mischen. Zur Erleichterung der Mischvorgänge kann man in allen Fällen eine (gegebenenfalls weitere) Menge Hilfslösemittel (G) zusetzen. Die Blockierung findet, je nach Blockierungsmittel bei Raumtemperatur oder zweckmäßig bei erhöhter Temperatur von 40 bis 100°C statt. Die Blokkierungsreaktion führt zu einem temperaturabhängigen Gleichgewicht. Durch orientierende Versuche läßt sich für ein gegebenes Polyisocyanatgemisch (C) und (D) und ein gegebenes Blockierungsmittel (F) die optimale Temperatur unschwer ermitteln. Die Menge an Blockierungsmittel (F) richtet sich nach der Anzahl der zu blokkierenden Isocyanatfunktionen. Zweckmäßig wendet man stöchiometrische Mengen oder einen leichten Unterschuß an Blockierungsmittel (F) an. um eine vollständige Umsetzung des Blockierungsmittels zu gewährleisten, so daß weder das Produkt, noch das wiederzuverwendende Hilfslösemittel (G) mit Blockierungsmittel (F) verunreinigt wird.

Grundsätzlich ist es auch möglich, zunächst hydrophobes Polyisocyanat (C) teilweise zu blockieren, dann die verbliebenen Isocyanatgruppen mit dem Hydrophilierungsmittel (E) umzusetzen, gegebenenfalls potentiell hydrophile Gruppen durch Neutralisieren in ionisch hydrophile Gruppen umzuwandeln und das so erhaltene blockierte hydrophil modifizierte Polyisocyanat (A) mit blockiertem hydrophoben Polyisocyanat (B) zu mischen. Dies ist aber nicht vorteilhaft, weil die Hydrophilierungsreaktion wegen der geringeren Isocyanatgruppen-Konzentration unwirtschaftlich lange Zeiten erfordert.

Wenn das Hydrophilierungsmittel (E) zunächst nur potentiell hydrophile Gruppen eingeführt hat, wird die Lösung der blockierten Polyisocyanate (A) und (B), gegebenenfalls nach Zusatz von weiterem Hilfslösemittel (G), neutralisiert, um die potentiell hydrophile Gruppen in ionisch hydrophile Gruppen umzuwandeln. Wie erwähnt, ist es in der Regel empfehlenswert, auch stärker saure und daher hydrophile Gruppen zu neutralisieren. Man wendet in beiden Fällen das Neutralisierungsmittel (H) in stöchiometrischen Mengen oder auch im Unter- oder Überschuß an. Im allgemeinen setzt man 50 bis 130 Mol-% der für eine vollständige Neutralisierung benötigten Menge ein. Durch die Menge des Neutralisierungsmittel läßt sich die Viskosität der Dispersion beeinflussen. Je größer der Unterschuß, um so weniger viskos ist die Dispersion. Andererseits fördern die ionischen hydrophilen Gruppen, die durch die Neutralisation entstehen, die Dispergierung der blockierten Polyisocyanate, so daß man nicht weniger als 50 Mol-% Neutralisierungsmittel verwenden sollte. Die optimale Menge hängt auch von dem Mengenverhältnis von blokkiertem hydrophil modifizierten Polyisocyanat (A) zu blockiertem hydrophoben Polyisocyanat (B) ab und läßt sich für eine bestimmte Dispersion durch Versuche unschwer ermitteln.

Zur Herstellung hilfslösemittelhaltiger wäßriger Dispersionen wird dann die neutralisierte Lösung, nach und nach oder auf einmal, mit Wasser versetzt, das zweckmäßig deionisiert ist. Alternativ wird die neutralisierte Lösung, wiederum nach und nach oder auf einmal, in zweckmäßig deionisiertes Wasser eingetragen. In jedem Fall sorgt man für intensive Mischung, mit oder ohne Scherkrafteinwirkung, wodurch die Ausbildung feinteiliger, stabiler Dispersionen begünstigt wird. Intensive Mischung erreicht man z.B. mit einem Propellerrührer oder einem Dissolver. Es ist auch möglich, die Teilschritte Neutralisierung und Dispergierung zusammenzufassen, beispielsweise indem man das Neutralisierungsmittel (H) und das Wasser gleichzeitig der Lösung der blockierten Polyisocyanate (A) und (B) zufügt, wobei das Neutralisierungsmittel (H) ganz oder teilweise in dem Wasser gelöst sein kann.

Die Menge des Wassers wird zweckmäßig so bemessen, daß sich eine Dispersion mit 35 bis 80 Gew.-% Feststoffanteil und kontinuierlicher wäßrig-hilfslösemittelhaltiger Phase ausbildet. Sie hängt von der Art und der Menge des Hilfslösemittels (G) sowie von der Art, dem Mengenverhältnis und dem Hydrophilierungsgrad der Polyisocyanate (A) und (B) ab und läßt sich durch Versuche unschwer ermitteln. Der Anteil des Hilfslösemittels an der Dispersion beträgt in der Regel weniger als 25 Gew.-%.

Die so erhältlichen hilfslösemittelhaltigen Dispersionen haben gegenüber den konventionellen, wasserfreien Systemen den Vorteil eines geringeren Lösemittelgehalts. Im Vergleich mit wäßrig-hilfslösemittelhaltigen Systemen mit externen Emulgatoren oder Schutzkolloiden sind sie vorteilhaft, weil die letztendlich erzeugten Lackfilme deutlich besser beständig gegen Feuchtigkeit und Witterungseinflüsse sind. Gegenüber wäßrig-hilfslösemittelhaltigen Systemen mit weitergehend oder vollständig hydrophilierten Polyisocyanaten weisen die erfindungsgemäßen Dispersionen denselben Vorteil auf; zudem sind sie erheblich weniger viskos, was ihre Verarbeitung erleichtert.

Besonders erwünscht sind die praktisch hilfslösemittelfreien Dispersionen mit 35 bis 60 Gew.-%, vorteilhaft 40 bis 50 Gew.-% Feststoffanteil, die man bei der erwähnten bevorzugten Ausführungsform des Verfahrens nach der Erfindung erhält. Dazu wird die Gesamtmenge an Wasser in zwei Teilmengen aufgeteilt. Zunächst wird der neutralisierten Lösung der blockierten Polyisocyanate (A) und (B) eine erste Teilmenge zugesetzt. Diese wird so bemessen, daß bei der Entfernung des Hilfslösemittels (G) keine Phasenumkehr stattfindet, das Wasser also in der kontinuierlichen Polyisocyanatphase gelöst bzw. dispergiert bleibt. Kennzeichnend für die Phasenumkehr ist ein starker Abfall der Viskosität. Die maximale erste Teilmenge Wasser, die noch nicht zur Phasenumkehr führt, hängt im wesentlichen von dem angestrebten Feststoffanteil der Dispersion sowie von der Art der Polyisocyanate, deren Mengenverhältnis und deren Hydrophilierungsgrad ab und läßt sich durch Versuche unschwer ermitteln. Sie beträgt in der Regel nicht mehr als 75% der Gesamtmenge an Wasser. Die optimale erste Teilmenge Wasser liegt im allgemeinen bei 30 bis 60% der Gesamtmenge. Die erste Teilmenge Wasser kann, ebenso wie die Lösung der Polyisocyanate (A) und (B), Raumtemperatur, mäßig erhöhte Temperatur, wie 30 bis 40°C, oder auch eine höhere Temperatur haben.

Nach der Zugabe des ersten Teilmenge Wasser erhöht man die Temperatur, rasch oder auch allmählich, bei Normaldruck oder vermindertem Druck auf 50 bis 110°C, so daß Hilfslösemittel (G) abdestilliert. Dabei sorgt man man wiederum für intensive Mischung, wie zuvor beschrieben. Dadurch fördert man die Ausbildung einer feinteiligen, stabilen Dispersion und erreicht zudem eine schnelle und weitgehende Entfernung des Hilfslösemittels (G). "Weitgehende Entfernung" bedeutet, daß der Gehalt der fertigen Dispersion an Hilfslösemittel (G) nach Zugabe der zweiten Teilmenge Wasser nicht mehr als 5 Gew.-% beträgt. Unter vermindertem Druck kann man dann weiteres Hilfslösemittel (G) entfernen, so daß dessen Gehalt in der gebrauchsfertigen Dispersion <2 Gew.-%, vorteilhaft <0.5 Gew.-% beträgt. Praktischer ist es allerdings, das Hilfslösemittel (G) schon vor dem Zusatz der zweiten Teilmenge Wasser so weitgehend zu entfernen, daß dessen Gehalt in der fertigen Dispersion, also schon nach dem Zusatz der zweiten Teilmenge Wasser, <2 Gew.-%, vorteilhaft <0,5 Gew.-% beträgt.

Wenn das Hilfslösemittel weitgehend entfernt ist, setzt man nach und nach, anteilsweise oder kontinuierlich, die zweite Teilmenge Wasser zu. Hierbei sorgt man zumindest bis zur Phasenumkehr für intensive Mischung, wie zuvor beschrieben, damit die entstehende Dispersion hinreichend feinteilig und stabil wird. Die Temperatur des restlichen Wassers liegt vorteilhaft in dem Bereich, in dem die Temperatur der Dispersion liegt, während das Hilfslösemittel (G) entfernt wird, zweckmäßig bei 60 bis 100°C. Wenn man bei der Zugabe der zweiten Teilmenge Wasser unter erhöhtem Druck arbeitet, kann die Temperatur des Wassers 100°C überschreiten. Dies erleichtert den Dispergiervorgang insbesondere bei relativ hochviskosen Systemen. Man muß allerdings dabei die Deblockierungstemperaturen beachten. Nach Zugabe der zweiten Teilmenge Wasser läßt man die Dispersion unter Rühren erkalten.

Man erhält stabile und feststoffreiche wäßrige, praktisch hilfslösemittelfreie Dispersionen, die bei Lagerung nach 6 Monaten bei Raumtemperatur oder nach 4 bis 8 Wochen bei 60°C in der Regel weniger als 1 Masse-% des darin enthaltenen Feststoffs abscheiden. Diese sowie etwaige größere Feststoffanteile, die sich gelegentlich bei nicht optimalen Bedingungen abscheiden, lassen sich durch Einwirkung entsprechend hoher Scherkräfte schnell und nachhaltig redispergieren. Gegenüber wäßrigen, praktisch hilfslösemittelfreien Dispersionen, die mit Emulgatoren oder Schutzkolloiden hergestellt wurden, haben die erfindungsgemäßen Dispersionen den Vorteil, daß die letztendlich hergestellten Lackfilme beständiger gegen Feuchtigkeit und Witterungseinflüsse sind. Das gilt auch im Vergleich mit entsprechenden Dispersionen, in denen stärker oder vollständig hydrophilierte Polyisocyanate vorliegen; diese haben zudem eine erheblich höhere Viskosität, was ihre Verarbeitung erschwert.

### 2.8 Verwendung der Dispersionen

Die stabilen und feststoffreichen wäßrigen und hilfslösemittelhaltigen oder praktisch hilfslösemittelfreien Dispersionen nach der Erfindung eignen sich als Vernetzungsmittel für heißvernetzende, lagerstabile Einkomponenten-Beschichtungssysteme, vorzugsweise zur Herstellung von umweltverträglichen wasserverdünnbaren Polyurethan-Beschichtungssystemen durch Kombination mit wäßrigen Lackharzen, d.h. wäßrigen Lösungen, Dispersionen bzw. Emulsionen und/oder anderen wasserverdünnbaren Systemen aus polymeren Harzen, mit im Mittel mehr als 1,5 NCO-reaktiven Gruppen, wie Hydroxyl- oder Aminogruppen, in jedem Molekül. Dazu werden die Dispersionen nach der Erfindung mit den wäßrigen Lackharzen vereinigt, zweckmäßig in solchen Mengen, daß auf jede NCO-Gruppe des Polyisocyanats eine NCO-reaktive Gruppe des Lackharzes entfällt. Gegebenenfalls werden dem Fachmann bekannte weitere Vernetzer, wie Melaminharze, und/oder bekannte Hilfsmittel, z.B. den Verlauf, den Glanz und/oder die Haftung fördernde Zusätze, zugemischt, und der fertige Lack wird, gegebenenfalls nach Verdünnung mit Wasser zur Regelung der Viskosität, in üblicher Weise auf das Substrat aufgebracht. Die Beschichtung wird zweckmäßig zunächst getrocknet und dann bei 100 bis 250°C unter Abspaltung des Blockierungsmittels eingebrannt, wodurch das Lackharz vernetzt wird.

### 3. Beispiele

Die folgenden Beispiele sollen die Erfindung weiter erläutern, aber nicht ihren Anwendungsbereich beschränken.

### Beispiel 1

### 1.1 Herstellung von hydrophilem Polyisocyanat (D)

741 g IPDI-Isocyanurat (VESTANAT^{(R)}T der Hüls AG) und 222 g IPDI (VESTANAT^{(R)}T1890 der Hüls AG) werden unter Rühren in 500 g Aceton gelöst. Man gibt unter Rühren 22,0 g einer 10 %-igen Lösung von Dibutylzinndilaurat in Aceton als Katalysator und 134 g pulverisierte Dimethylolpropionsäure hinzu und erwärmt das Gemisch auf etwa 60°C, so daß es am Rückfluß siedet. Man rührt, bis die Dimethylolpropionsäure vollständig gelöst und der NCO-Gehalt der Lösung (bestimmt nach DIN 53185) auf 7,8 bis 8,0 % abgesunken ist, was 6 bis 8 Stunden in Anspruch nimmt.

### 1.2 Zugabe des hydrophoben Polyisocyanats (C) und Blockierung

In die gerade nicht mehr siedende Lösung nach Ziffer 1.1 werden unter Rühren 1.482 g IPDI-Isocyanurat (siehe Ziffer 1.1) und zur Verdünnung 3.000 g Aceton gegeben. Man rührt das Gemisch bei 50 bis 60°C so lange, bis eine homogene Lösung entsteht, kühlt diese auf 40 bis 55 °C ab und fügt unter Kühlung 783,0 g Methylethylketoxim in einer solchen Geschwindigkeit zu, daß die Temperatur im Bereich von 40 bis 50°C bleibt. Man rührt weitere 30 Minuten bei dieser Temperatur und kühlt dann unter Rühren auf 30°C ab.

### 1.3 Dispergierung der blockierten Polyisocyanate (A) und (B)

Zu der nach Ziffer 1.2 hergestellten Lösung gibt man 117 g N,N-Diethylaminoethanol und anschließend nach und nach und unter Rühren 1.700 g deionisiertes Wasser. Das Gemisch wird in einem offenen Gefäß auf 80 bis 95°C erhitzt. Wenn das Aceton nahezu vollständig verdampft ist, setzt man innerhalb von 5 bis 10 Minuten praktisch kontinuierlich unter intensivem Mischen mittels eines Propellerrührers weitere 2.700 g auf 70 bis 85°C erwärmtes deionisiertes Wasser zu. Dann läßt man die entstandene Dispersion unter Rühren auf 30°C abkühlen.

Man erhält eine weiße Dispersion mit einem Feststoffgehalt von 45 Gew.-% (nach DIN 53 216 bei 105°C), einem Acetongehalt von 0,3 Gew.-% (nach Headspace-GC), einem pH-Wert von 8,9 (nach DIN 53 785) und einer Viskosität bei Raumtemperatur von 60 mPa.s (nach DIN 53 019 bei D = 200 s⁻¹). Bei Lagerung über 6 Monate bei Raumtemperatur bzw. über 4 Wochen bei 50°C erfolgt keine Koagulierung oder Sedimentation. Innerhalb dieser Zeit ist die Dispersion uneingeschränkt verwendbar.

### Beispiel 2

### 2.1 Herstellung von hydrophilem Polyisocyanat (A), Zugabe des hydrophoben Isocyanats (C) und Blockierung

Man stellt das hydrophile Polyisocyanat her, wie in Ziffer 1.1 beschrieben, und setzt der gerade nicht mehr siedenden Lösung unter Rühren 741 g IPDI-Isocyanurat sowie 222 g IPDI (wie in Beispiel 1) und zur Verdünnung 1.368 g Aceton zu. Man rührt bei 50 bis 60°C so lange weiter, bis eine homogene Lösung entsteht, kühlt diese auf 40 bis 55°C ab und gibt 696 g Methylethylketoxim in einer solchen Geschwindigkeit hinzu, daß die Temperatur im Bereich von 40 bis 50°C bleibt. Man rührt das Gemisch weitere 30 Minuten bei dieser Temperatur und läßt es dann unter Rühren auf 30°C abkühlen.

### 2.2 Dispergierung der blockierten Polyisocyanate (A) und (B)

Zu der nach Ziffer 2.1 hergestellten Lösung gibt man 80 g N,N-Dimethylaminoethanol und anschließend 1.484 g deionisiertes Wasser. Das Gemisch wird unter intensivem Mischen auf 75 bis 85°C erhitzt und durch Absenken des Druckes auf 800 bis 900 mbar weitgehend von Aceton befreit. Dann setzt man unter weiterem intensivem Mischen in kurzen Abständen viermal 470 g auf 70 bis 80°C vorgewärmtes Wasser langsam zu und läßt die entstandene Dispersion unter Rühren auf 30°C abkühlen.

Man erhält eine weiße Dispersion mit einem Feststoffgehalt von 44 Gew.-% (nach DIN 53 216 bei 105°C), einem Acetongehalt von 0,3 Gew.-% (nach Headspace-GC), einem pH-Wert von 8,8 (nach DIN 53 785) und einer Viskosität von 50 mPa·s (nach DIN 53 019 bei D = 200 s⁻¹). Bei Lagerung über 6 Monate bei Raumtemperatur oder über 4 Wochen bei 50°C erfolgt keine Koagulierung oder Sedimentation. Innerhalb dieser Zeiten ist die Dispersion uneingeschränkt verwendbar.

### Beispiel 3

### 3.1 Herstellung von hydrophilem Polyisocyanat (D)

1.482 g IPDI-Isocyanurat (VESTANAT^{(R)}T 1890 der Hüls AG) werden unter Rühren in 1.000 g Aceton gelöst. Man gibt unter Rühren 3,2 g Dibutylzinndilaurat als Katalysator und 134 g pulverisierte Dimethylolpropionsäure hinzu und erwärmt das Gemisch auf etwa 60°C, so daß es am Rückfluß siedet. Man rührt, bis die Dimethylolpropionsäure vollständig gelöst und der NCO-Gehalt der Lösung (nach DIN 53 185) auf 6,1 bis 6,3 % abgesunken ist, was 6 bis 8 Stunden in Anspruch nimmt.

### 32 Zugabe des hydrophoben Polyisocyanats (C) und Blockierung

In die gerade nicht mehr siedende Lösung nach Ziffer 3.1 werden unter Rühren 1.734 HDI-Isocyanurat (DESMODUR^{(R)}N 3300 der Bayer AG) und zur Verdünnung 2.500 g Aceton gegeben. Man rührt das Gemisch bei 50 bis 60°C so lange, bis eine homogene Lösung entsteht. kühlt diese auf 40 bis 55°C ab und fügt unter Kühlung 1.131 g Methylethylketoxim in einer solchen Geschwindigkeit zu, daß die Temperatur im Bereich von 40 bis 50°C bleibt. Man rührt weitere 30 Minuten bei dieser Temperatur und kühlt dann die Lösung der blockierten Polyisocyanate auf 30°C ab.

### 3.3 Dispergierung der blockierten Polyisocyanate (A) und (B)

Zu der nach Ziffer 3.2 hergestellten Lösung gibt man 89 g N,N-Dimethylaminoethanol und anschließend nach und nach und unter starkem Rühren 2.000 g deionisiertes Wasser. Das Gemisch wird unter intensivem Mischen auf 75 bis 90°C erhitzt und durch Absenken des Drukkes auf 800 bis 900 mbar weitgehend von Aceton befreit. Dann setzt man innerhalb von 10 bis 20 Minuten unter weiterem intensiven Mischen praktisch kontinuierlich weitere 4.200 g auf 80 bis 90°C erwärmtes deionisiertes Wasser zu.

Man erhält eine weiße Dispersion mit einem Feststoffgehalt von 42 Gew.-% (nach DIN 53 216 bei 105°C), einem Acetongehalt von 0,4 Gew.-% (nach Headspace-GC), einem pH-Wert von 8,9 (nach DIN 53 785) und einer Viskosität von 250 mPa·s (nach DIN 53 019 bei D = 200 s⁻¹). Bei Lagerung der Dispersion über einen Zeitraum von 6 Monaten bei Raumtemperatur bzw. von 4 Wochen bei 50°C erfolgt keine Koagulierung oder Sedimentation. Während dieser Zeiträume ist die Dispersion uneingeschränkt verwendbar.

### Beispiel 4 (nicht erfindungsgemäßes Vergleichsbeispiel)

Man verfährt zunächst, wie unter den Ziffern 1.1 und 1.2 beschrieben und setzt der Lösung der blokkierten Polyisocyanate (A) und (B) 89 g N.N-Dimethylaminethanol und anschließend 4.112 g auf 30 bis 40 erwärmtes deionisiertes Wasser auf einmal zu. Die Mischung wird unter intensivem Mischen bei 50 bis 60°C im Vakuum weitgehend von Aceton befreit. Dabei treten schon bald Inhomogenitäten und Feststoffausfällungen ein. Letztlich tritt vollständige Phasentrennung ein. Die abgeschiedene weiße pastöse Masse ist nicht redispergierbar.

## Patentansprüche

1. Verfahren zur Herstellung stabiler und feststoffreicher wässriger Dispersionen von blockierten Isocyanaten, die praktisch hilfslösemittelfrei sind,
**dadurch gekennzeichnet,**
**dass** man
(1) eine Lösung eines Polyisocyanatgemisches aus 20 bis 70 Gew.-% eines blockierten hydrophil modifizierten Polyisocyanats (A) und 30 bis 80 Gew.-% eines blockierten hydrophoben Polyisocyanats (B) in einem Hilfslösemittel (G) herstellt und
(2) der Lösung der Stufe (1) eine erste Teilmenge des insgesamt verwendeten Wassers zusetzt,
(3) das Hilfslösemittel (G) unter intensivem Mischen aus dem entstehenden wässrigen, hilfslösemittelhaltigen Gemisch weitgehend entfernt und
(4) dem verbleibenden Gemisch aus den blockierten Polyisocyanaten (A) und (B) und der ersten Teilmenge Wasser eine zweite Teilmenge Wasser zusetzt,
mit der Maßgabe, dass (i) die erste Teilmenge Wasser so bemessen wird, dass in dem Gemisch, das nach der weitgehenden Entfernung des Hilfslösemittels (G) verbleibt, die Polyisocyanate (A) und (B) die kontinuierliche Phase bilden und (ii) die Zugabe der zweiten Teilmenge Wasser zumindest bis zur Phasenumkehr unter intensivem Mischen erfolgt.

2. Verfahren nach Anspruch 1,
**dadurch gekennzeichnet,**
**dass** das blockierte hydrophil modifizierte Polyisocyanat (A) ein blockiertes ionisch hydrophil modifiziertes Polyisocyanat ist, das durch vollständiges oder teilweises Neutralisieren eines Polyisocyanats mit einer potentiell hydrophilen Gruppe entsteht.

3. Verfahren nach Anspruch 1 oder 2,
**dadurch gekennzeichnet,**
**dass** die potentiell hydrophile Gruppe eine Carboxylgruppe und das Neutralisierungsmittel Ammoniak oder ein Amin ist.

4. Verfahren nach einem der Ansprüche 1 bis 3,
**dadurch gekennzeichnet,**
**dass** man das Gemisch der Polyisocyanate (A) und (B) dadurch erzeugt, dass man eine diesem Gemisch entsprechende Menge hydrophobes Polyisocyanat (C) nur teilweise hydrophiliert, indem man sie mit einer solchen Menge Hydrophilierungsmittel (E) umsetzt, dass die Anteile an entstehendem hydrophil modifizierten Polyisocyanat (D) und an verbliebenem hydrophoben Polyisocyanat (C) dem für die fertige Dispersion angestrebten Verhältnis von blockierten hydrophil modifizierten Polyisocyanat (A) zu blockiertem hydrophoben Polyisocyanat (B) entsprechend, gegebenenfalls potentiell hydrophile Gruppen durch Neutralisieren in ionisch hydrophile Gruppen überführt und die nicht umgesetzten Isocyanatgruppen des Hydrophilierungsgemisches blockiert.

5. Stabile und feststoffreiche wässrige, praktisch hilfslösemittelfreie Dispersion,
**gekennzeichnet durch** ein Polyisocyanatgemisch aus 20 bis70 Gew.-% eines blockierten hydrophil modifizierten Polyisocyanats (A) und 30 bis 80 Gew.-% eines blockierten hydrophoben Polyisocyanats (B) als disperse Phase, einen Feststoffgehalt von 40 bis 60 Gew.% und einen Gehalt an Hilfslösemittel (G) von < 2 Gew.-%

6. Dispersion nach Anspruch 5,
**gekennzeichnet durch** einen Gehalt an Hilfslösemittel (G) von < 0,5 Gew.-%.

7. Dispersion nach Anspruch 5 oder 6,
**dadurch gekennzeichnet,**
**dass** das blockierte hydrophil modifizierte Polyisocyanat (A) ein blockiertes ionisch hydrophil modifiziertes Polyisocyanat ist, das durch vollständiges oder teilweises Neutralisieren eines Polyisocyanats mit einer potentiell hydrophilen Gruppe entsteht.

8. Dispersion nach Anspruch 7,
**dadurch gekennzeichnet,**
**dass** die potentiell hydrophile Gruppe eine Carboxylgruppe und das Neutralisierungsmittel (H) Ammoniak oder ein Amin ist.

9. Verwendung der Dispersionen nach einem der Ansprüche 5 bis 8 als Vernetzungsmittel für Lackharze.

## Claims

1. A process for preparing a stable and high-solids aqueous dispersion of blocked isocyanates which is virtually free from auxiliary solvent,
**characterized in that**
(1) a solution of a polyisocyanate mixture of from 20 to 70 % by weight of a blocked, hydrophilically modified polyisocyanate (A) and from 30 to 80 % by weight of a blocked, hydrophobic polyisocyanate (B) is prepared in an auxiliary solvent (G), and
(2) a first portion of the total amount of water used is added to the solution of stage (1),
(3) the auxiliary solvent (G) is substantially removed from the aqueous mixture that is formed, containing auxiliary solvent, with intensive mixing, and
(4) a second portion of water is added to the remaining mixture comprising the blocked polyisocyanates (A) and (B) and the first portion of water,
with the provisos that (i) the first portion of water is of a size such that the polyisocyanates (A) and (B) form the continuous phase in the mixture which remains after the substantial removal of the auxiliary solvent (G), and (ii) the addition of the second portion of water takes place with intensive mixing, at least up until the point of phase inversion.

2. A process according to claim 1, **characterized in that** the blocked, hydrophilically modified polyisocyanate (A) is a blocked, ionically hydrophilically modified polyisocyanate which forms as a result of complete or partial neutralization of a polyisocyanate having a potentially hydrophilic group.

3. A process according to claim 1 or 2, **characterized in that** the potentially hydrophilic group is a carboxyl group and the neutralizing agent is ammonia or an amine.

4. A process according to any one of claims 1 to 3, **characterized in that** the mixture of the polyisocyanates (A) and (B) is produced by only partially hydrophilicizing an amount of hydrophobic polyisocyanate (C) corresponding to this mixture by reacting it with an amount of hydrophilicizing agent (E) which is such that the proportions of resultant, hydrophilically modified polyisocyanate (D) and of remaining hydrophobic polyisocyanate (C) correspond to the desired ratio of blocked hydrophilically modified polyisocyanate (A) to blocked hydrophobic polyisocyanate (B) for the finished dispersion, by converting any potentially hydrophilic groups by neutralization into ionically hydrophilic groups and by blocking the unreacted isocyanate groups of the hydrophilicizing mixture.

5. A stable and high-solids aqueous dispersion which is virtually free from auxiliary solvent, **characterized by** a polyisocyanate mixture of from 20 to 70 % by weight of a blocked, hydrophilically modified polyisocyanate (A) and from 30 to 80 % by weight of a blocked, hydrophobic polyisocyanate (B) as disperse phase, a solids content of from 40 to 60 % by weight and a content of auxiliary solvent (G) of <2 % by weight.

6. A dispersion according to claim 5, **characterized by** a content of auxiliary solvent (G) of <0.5 % by weight.

7. A dispersion according to claim 5 or 6, **characterized in that** the blocked, hydrophilically modified polyisocyanate (A) is a blocked, ionically hydrophilically modified polyisocyanate which forms by complete or partial neutralization of a polyisocyanate having a potentially hydrophilic group.

8. A dispersion according to claim 7, **characterized in that** the potentially hydrophilic group is a carboxyl group and the neutralizing agent (H) is ammonia or an amine.

9. The use of a dispersion according to any one of claims 5 to 8 as a crosslinking agent for film-forming resins.

## Revendications

1. Procédé de production de dispersions aqueuses, stables et à haute teneur en matières solides, d'isocyanates bloqués, qui sont pratiquement exemptes de solvants auxiliaires,
**caractérisé en ce qu'**
l'on
(1) on produit une solution d'un mélange de polyisocyanates composé de 20 à 70 % en poids d'un polyisocyanate (A) modifié hydrophile bloqué, et de 30 à 80 % en poids d'un polyisocyanate (B) hydrophobe bloqué, dans un solvant auxiliaire (G),
(2) on ajoute à la solution de l'étape (1) une première quantité partielle de l'eau utilisée au total,
(3) on élimine largement le solvant auxiliaire (G), sous mélange intensif, du mélange aqueux obtenu, contenant le solvant auxiliaire, et
(4) on ajoute une deuxième quantité partielle d'eau au mélange restant, composé des polyisocyanates (A) et (B) bloqués et de la première quantité partielle d'eau,
à la condition que (i) la première quantité partielle d'eau est dimensionnée de façon que, dans le mélange qui reste après la large élimination du solvant auxiliaire (G), les polyisocyanates (A) et (B) forment la phase continue et (ü) l'addition de la seconde quantité partielle d'eau est réalisée sous mélange intensif au moins jusqu'à l'inversion de phase.

2. Procédé selon la revendication 1,
**caractérisé en ce que**
le polyisocyanate (A) modifié hydrophile bloqué, est un polyisocyanate modifié hydrophile ionique bloqué, obtenu par neutralisation complète ou partielle d'un polyisocyanate avec un groupe potentiellement hydrophile.

3. Procédé selon la revendication 1 ou 2,
**caractérisé en ce que**
le groupe potentiellement hydrophile est un groupe carboxyle et le moyen de neutralisation est de l'ammoniac ou une amine.

4. Procédé selon l'une des revendications 1 à 3,
**caractérisé en ce que**
le mélange des polyisocyanates (A) et (B) est produit en hydrophilisant seulement en partie une quantité de polyisocyanate (C) hydrophobe correspondant à ce mélange, en la mettant en réaction avec une quantité de moyen d'hydrophilisation (E) telle que les proportions de polyisocyanate (D) modifié hydrophile obtenu et de polyisocyanate (C) hydrophobe restant correspondent au rapport voulu, pour la dispersion finie, de polyisocyanate (A) modifié hydrophile bloqué au polyisocyanate (B) bloqué hydrophobe, en transformant le cas échéant les groupes potentiellement hydrophiles par neutralisation en groupes hydrophiles de façon ionique, et en bloquant les groupes isocyanates non entrés en réaction du mélange d'hydrophilisation.

5. Dispersion aqueuse stable et à haute teneur en matières solides, pratiquement exempte de solvant auxiliaire,
**caractérisée par**
un mélange de polyisocyanates composé de 20 à 70 % en poids d'un polyisocyanate (A) modifié hydrophile bloqué, et de 30 à 80 % en poids d'un polyisocyanate (B) bloqué hydrophobe en tant que phase dispersée, avec une teneur en matières solides de 40 à 60 % en poids et une teneur en solvant auxiliaire (G) < 2 % en poids.

6. Dispersion selon la revendication 5,
**caractérisée par**
une teneur en solvant auxiliaire (G) < 0,5 % en poids.

7. Dispersion selon la revendication 5 ou 6,
**caractérisée en ce que**
le polyisocyanate (A) modifié hydrophile bloqué, est un polyisocyanate bloqué modifié hydrophile ionique, qui est obtenu par neutralisation complète ou partielle d'un polyisocyanate avec un groupe potentiellement hydrophile.

8. Dispersion selon la revendication 7,
**caractérisée en ce que**
le groupe potentiellement hydrophile est un groupe carboxyle et le moyen de neutralisation (H) est de l'ammoniac ou une amine.

9. Utilisation des dispersions selon l'une des revendications 5 à 8, comme réticulant pour résines de vernis.
